# EUROPEAN PATENT SPECIFICATION

(11) **EP 2 266 484 B1**
(45) Date of publication and mention of the grant of the patent: **07.05.2014**
(21) Application number: 09730638.5
(22) Date of filing: 17.02.2009
(51) Int. Cl.: A61B 18/02, A61B 5/04, A61B 18/00, A61B 5/00, A61B 17/00

(54) **NEEDLE PROBE FOR COOLING OPERATION AND COOLING OPERATION SYSTEM**
NADELSONDE FÜR KÜHLUNGSVORGÄNGE UND KÜHLUNGSVORGANGSSYSTEM
SONDE AIGUILLE POUR OPÉRATION DE REFROIDISSEMENT ET SYSTÈME D'OPÉRATION DE REFROIDISSEMENT

(30) Priority: 11.04.2008 JP 2008102974; 22.11.2008 JP 2008298861
(43) Date of publication of application: 29.12.2010
(73) Proprietor: National University Corporation Kyushu Institute Of Technology, Tobata-ku Kitakyushu-shi Fukuoka 804-8550 (JP)
(72) Inventor: YAMAKAWA, Takeshi, Kitakyushu-shi Fukuoka 804-8550 (JP); ZIMIN, Lev Grigorievich, Kitakyushu-shi Fukuoka 804-8550 (JP); ZIMIN, Yury L'vovich, Kitakyushu-shi Fukuoka 804-8550 (JP)
(74) Representative: Giles, Ashley Simon
(86) International application number: PCT/JP2009/000639
(87) International publication number: WO 2009/125535

(56) References cited:
- JP-A- 60 137 359
- JP-T- 9 511 414
- JP-T- 2002 513 614
- US-A1- 2008 065 062

## Description

### Technical Field

The present invention relates to a cooling needle probe and cooling system.

### Background Art

Cryogenic operations which involve freezing an affected area are used widely.

A cryogenic operation, which freezes cells, thaws the frozen cells subsequently, and thereby destroys and necroses cell organelles, is considered suitable because of reduced surgical pain, postoperative scarring (alteration), postoperative complications, and surgical costs.

Cryogenic operations are broadly divided into two types according to affected areas to be operated on.

One of the types is used to treat affected areas such as the stomach which are affected by a cancer, tumor, or the like. In this case, a cooling probe is inserted to the affected area in the body, for example through the mouth, to freeze the affected area.

Cryogenic operations, which use a probe inserted into the body in this way, require utmost care. For example, it is required to accurately treat the affected area by precisely controlling heat transfer or thermally insulate the cryogenic probe until the probe reaches the affected area. Also, circulating a high-pressure cryogenic refrigerant through the probe poses a safety problem.

In view of these points, various cryosurgical probes and cryosurgical apparatuses have been proposed. However, none of the cryosurgical probes and cryosurgical apparatuses can avoid size increases when the safety measures and other necessary ancillary parts and ancillary devices are provided. In some cases, the Peltier effect is used for efficient cooling.

In operations performed by means of the cryosurgical probe and cryosurgical apparatus, cooling is controlled by manually interrupting circulation of the refrigerant (see, for example, Patent Documents 1 and 2).

The other type of cryogenic operation involves cooling a minute part rapidly by inserting a cooled probe to subcutaneous tissue through the skin. For example, it has recently been reported that there is a possibility that an epileptic seizure can be suppressed by local cooling in the brain.

For that purpose, under the present situation, the above-described probe used to treat the affected area in the body is used as it is, to cool, for example, an area of approximately 4 mm square on a brain surface.

Incidentally, cryosurgical probes with a built-in temperature sensor such as a thermocouple have been proposed, examples of which include a cryoneedle of a cryogenic surgical apparatus, where the cryoneedle has a diameter of less than 3.2 mm, specifically, in the range of approximately 0.8 mm to 2 mm, and incorporates a built-in thermocouple. A measuring junction of the thermocouple is placed at a location away from a distal part of the cryoneedle for proper functioning of the thermocouple.

Again, the temperature sensor is used only for temperature measurement and display, and magnitude of cooling is adjusted by manually operating a refrigerant unit (see Patent Document 3).

A further previously proposed cooled surgical probe with a temperature sensor is disclosed in US2008/0065062 A1.
Patent Document 1: National Publication of International
Patent No. 2002-513614
Patent Document 2: Japanese Patent Laid-Open No. 2006-130024
Patent Document 3: National Publication of International
Patent No. 2000-513963

### Disclosure of the Invention

### Problems to be Solved by the Invention

However, for example, in the epilepsy treatment described above, it will be more desirable if a more minute region can be cooled accurately. Also, if there is a probe which can accurately cool such a minute region, it will be suitable, for example, for sampling living cells.

The present invention has been made in view of the above problems and has an object to provide a cooling needle probe and cooling system which can accurately cool a minute region.

The invention is defined in the claims. Other embodiments are presented for illustrative purposes only.

### Means for Solving the Problems

The present invention provides a cooling needle probe comprising a first cylindrical body and a second cylindrical body which are made of thin-walled dissimilar metals and run longitudinally by being electrically insulated from each other, where the second cylindrical body is installed around the first cylindrical body, wherein the first cylindrical body and the second cylindrical body are closed and joined at one end, forming a distal part which electrically interconnects the first cylindrical body and the second cylindrical body; a minute communicating section is formed near the distal part to communicate between interior and exterior of the first cylindrical body; wherein the cooling needle probe is configured such that a refrigerant flowing into one of: a cavity between the second cylindrical body and the first cylindrical body; and a cavity in the first cylindrical body, evaporates or adiabatically expands upon passage through the minute communicating section, thereby generating cold, which flows into the other cavity to cool the distal part; and the distal part serves as a measuring junction of a thermocouple while the first cylindrical body and the second cylindrical body serve as compensating leads of the thermocouple.

In the cooling needle probe according to the present invention, preferably a combination of materials for the first cylindrical body and the second cylindrical body is selected from among a combination of Chromel and Alumel, a combination of Chromel and copper, a combination of Chromel and Constantan, a combination of stainless steel grade 304 and Kovar, a combination of Constantan and copper, and a combination of platinum and platinum-rhodium.

Also, in the cooling needle probe according to the present invention, preferably the probe is 1 mm or less in diameter.

Also, the present invention provides a cooling system comprising: the cooling needle probe; a probe grip installed on a side opposite the distal part of the cooling needle probe; a refrigerant supply and discharge mechanism adapted to supply a refrigerant to the cooling needle probe and discharge the refrigerant after cold is released; and a refrigerant quantity control mechanism adapted to control a supply quantity of the refrigerant based on a signal from the thermocouple.

In the cooling needle probe according to the present invention, preferably the second cylindrical body which runs longitudinally by being electrically insulated from the first cylindrical body or surroundings is connected to a biomedical signal measuring mechanism or an electrical stimulus supply mechanism so as to be able to measure a biomedical signal or give an electrical stimulus to a living body, via the distal part.

Also, the present invention provides a cooling system comprising: the cooling needle probe; a probe grip installed on a side opposite the distal part of the cooling needle probe; a refrigerant supply and discharge mechanism adapted to supply a refrigerant to the cooling needle probe and discharge the refrigerant after cold is released; a refrigerant quantity control mechanism adapted to control a supply quantity of the refrigerant based on a signal from the thermocouple; and a biomedical signal measuring mechanism or an electrical stimulus supply mechanism connected with the first cylindrical body or the second cylindrical body.

Preferably, the cooling needle probe according to the present invention further comprises a first electrode unit which runs longitudinally by being electrically insulated from surroundings and has a tip in or near the distal part, characterized in that the first electrode unit can measure a biomedical signal via the tip and/or give an electrical stimulus to a living body via the tip by being connected to a biomedical signal measuring mechanism or an electrical stimulus supply mechanism or being connected to the biomedical signal measuring mechanism and the electrical stimulus supply mechanism in such a way as to be able to switch between the biomedical signal measuring mechanism and the electrical stimulus supply mechanism.

Also, the present invention provides a cooling system comprising: the cocling needle probe; a probe grip installed on a side opposite the distal part of the cooling needle probe; a refrigerant supply and discharge mechanism adapted to supply a refrigerant to the cooling needle probe and discharge the refrigerant after cold is released; a refrigerant quantity control mechanism adapted to control supply quantity of the refrigerant based on a signal from the thermocouple; characterized in that the first electrode unit includes a biomedical signal measuring mechanism and an electrical stimulus supply mechanism.

Preferably, the cooling needle probe according to the present invention further comprises a first electrode unit which runs longitudinally by being electrically insulated from surroundings and has a tip in or near the distal part, characterized in that one of the second cylindrical body which runs longitudinally by being electrically insulated from the first cylindrical body or surroundings and the first electrode unit can measure a biomedical signal from a predetermined area of a living body and/or give an electrical stimulus to the predetermined area of the living body, via the distal part or the tip, by being connected to a biomedical signal measuring mechanism or an electrical stimulus supply mechanism or being connected to the biomedical signal measuring mechanism and the electrical stimulus supply mechanism in such a way as to be able to switch between the biomedical signal measuring mechanism and the electrical stimulus supply mechanism, and the other of the two can measure a biomedical signal from an area other than the predetermined area by being connected to the biomedical signal measuring mechanism.

Preferably the cooling needle probe according to the present invention, further comprises a second electrode unit and a third electrode unit each of which runs longitudinally by being electrically insulated from surroundings and has a tip in or near the distal part, characterized in that the second electrode unit can measure a biomedical signal via the tip by being connected to a biomedical signal measuring mechanism; and the third electrode unit can give an electrical stimulus to a living body via the tip by being connected to an electrical stimulus supply mechanism.

Also, the present invention provides a cooling system comprising: the cooling needle probe; a probe grip installed on a side opposite the distal part of the cooling needle probe; a refrigerant supply and discharge mechanism adapted to supply a refrigerant to the cooling needle probe and discharge the refrigerant after cold is released; a refrigerant quantity control mechanism adapted to control supply quantity of the refrigerant based on a signal from the thermocouple; a biomedical signal measuring mechanism connected with the second electrode unit; and an electrical stimulus supply mechanism connected with a third electrode unit.

Preferably the cooling needle probe according to the present invention, further comprises a second electrode unit and a third electrode unit each of which runs longitudinally by being electrically insulated from surroundings and has a tip in or near the distal part, characterized in that one of the second electrode unit, the third electrode unit, and the second cylindrical body can measure a biomedical signal from a predetermined area of a living body via the tip by being connected to a biomedical signal measuring mechanism, where the second cylindrical body runs longitudinally by being electrically insulated from the first cylindrical body or surroundings; another of the three can give an electrical stimulus to the predetermined area of the living body via the tip by being connected to an electrical stimulus supply mechanism; and the remaining one can measure a biomedical signal from an area other than the predetermined area.

### Advantages of the Invention

With the cooling needle probe according to the present invention, since the first cylindrical body and the second cylindrical body are closed and joined at one end, forming a distal part which electrically interconnects the first cylindrical body and the second cylindrical body, with the distal part serving as a measuring junction of a thermocouple and with the first cylindrical body and the second cylindrical body serving as compensating leads (working leads) of the thermocouple, there is no need to incorporate a temperature sensor in the probe. This makes it possible to reduce the probe to a very small diameter which can accurately cool a minute region of, for example, a living body.

Also, with the cooling needle probe according to the present invention, since the second cylindrical body which runs longitudinally by being electrically insulated from the first cylindrical body or surroundings is connected to a biomedical signal measuring mechanism or an electrical stimulus supply mechanism so as to be able to measure a biomedical signal via the distal part or give an electrical stimulus to a living body via the distal part, it is possible to measure, for example, brain waves in a minute region of the human brain to be cooled accurately and determine accurate position and character of, for example, an epileptogenic zone in the minute region without the need to provide a special electrode unit to measure a biomedical signal or give an electrical stimulus to a living body. Also, it is possible to observe, for example, responses of the minute region in the human brain to be cooled accurately to electrical stimuli, making it easy to confirm the minute region to be cooled or position the probe accurately in the minute region to be cooled. This also makes it possible to carry out a cooling operation efficiently.

Also, since the cooling needle probe according to the present invention further comprises the first electrode unit which runs longitudinally by being electrically insulated from the surroundings and has a tip in or near the distal part, or further comprises the second electrode unit and the third electrode unit, the cooling needle probe can more properly measure a biomedical signal or give an electrical stimulus to a living body using these electrode units exclusively for the electrical stimulus supply mechanism or the like. Furthermore, using one of the electrode units for biomedical signal measurement, e.g., as an electrode for measurement of a reference potential, the cooling needle probe can easily measure the biomedical signal without providing an electrode for use to measure the reference potential separately from the probe.

Also, being equipped with the cooling needle probe, cooling system according to the present invention can take good advantage of the cooling needle probe.

### Brief Description of the Drawings

Figure 1 is a diagram showing a schematic configuration of a cooling needle probe according to a first example of an embodiment of the present invention;
Figure 2 is an expanded view showing a distal portion of the probe shown in Figure 1;
Figure 3 is a side view of a distal portion for illustrating a schematic configuration of a cooling needle probe according to a third example of the present embodiment;
Figure 4 is a front view of a distal portion of the probe shown in Figure 3;
Figure 5 is a side view of a distal portion for illustrating a schematic configuration of a cooling needle probe according to a fourth example of the present embodiment;
Figure 6 is a front view of a distal portion of the probe shown in Figure 5;
Figure 7 is a side view of a distal portion for illustrating a schematic configuration of a cooling needle probe according to a variation of the fourth example of the present embodiment;
Figure 8 is a front view of a distal portion shown in Figure 7;
Figure 9 is a diagram showing a schematic configuration of a cooling system according to a fifth example of the present embodiment;
Figure 10 is a diagram showing a schematic configuration of a cooling system according to a sixth example of the present embodiment;
Figure 11 is a diagram showing a schematic configuration of a cooling system according to a seventh example of the present embodiment; and
Figure 12 is a diagram showing a schematic configuration of a cooling system according to an eighth example of the present embodiment.

### Description of Symbols

10, 10a, 10b, 10c Cooling needle probe
12 Second cylindrical body
14 First cylindrical body
16 Insulating coating
18 Distal part
20 Minute communicating section
22 Cooling system
24 Probe grip
26 Refrigerant supply and discharge mechanism
28 Refrigerant quantity control mechanism
30 Discharge pipe
32 Supply pipe
34 Storage unit
36 First electrode unit
40, 40a Third electrode unit
42, 42a Second electrode unit
44 Biomedical signal measuring mechanism
46 Electrical stimulus supply mechanism

### Best Mode for Carrying Out the Invention

An embodiment of the present invention will be described below with reference to the drawings.

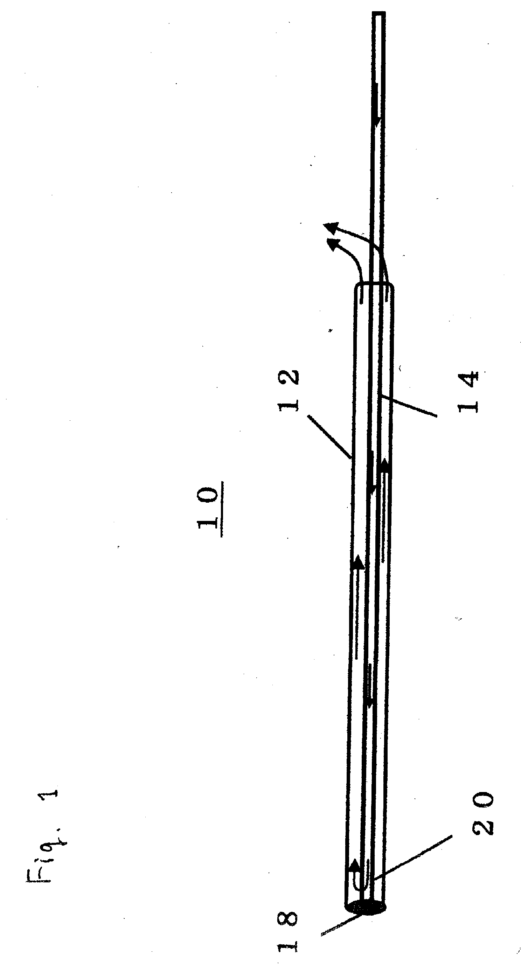

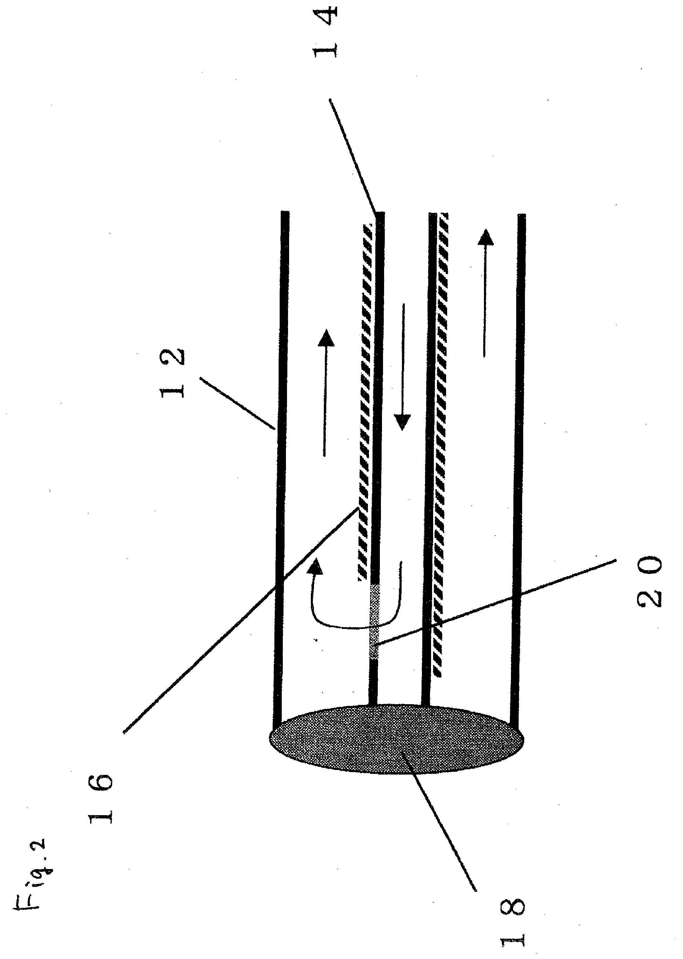

A cooling needle probe according to a first example of the present embodiment will be described with reference to Figures 1 and 2.

The cooling needle probe 10 according to the first example of the present embodiment, whose schematic configuration is shown in Figure 1 as an example, includes a first cylindrical body 14 and second cylindrical body 12.

The first cylindrical body 14 and second cylindrical body 12 are made of thin-walled dissimilar metals and run longitudinally by being electrically insulated from each other.

The first cylindrical body 14 and second cylindrical body 12 not only serve basic roles of a probe in which a refrigerant is circulated, but also serve as compensating leads (working leads) of a thermocouple. As long as these functions are served, any metal materials may be used as appropriate.

To allow measurements down to cryogenic temperatures, preferably the cylindrical body acting as a positive leg is made of nickel-chromium alloy (such as Chromel, which is a registered trademark), iron such as stainless steel grade 304, or copper while the cylindrical body acting as a negative leg is made of nickel-aluminum alloy (such as Alumel, which is a registered trademark), Constantan, or iron-nickel-cobalt alloy (such as Kovar, which is a registered trademark). On the other hand, if it is not necessary to take measurements at cryogenic temperatures, preferably the cylindrical body acting as a positive leg is made of platinum-rhodium while the cylindrical body acting as a negative leg is made of platinum.

From the standpoint of increasing strength of the second cylindrical body 12, preferably the second cylindrical body 12 is made of Chromel, stainless steel grade 304, Constantan, or platinum and the first cylindrical body 14 is made of a material which develops a thermal electromotive force relative to the material of the second cylindrical body 12. Preferably a combination of materials for the first cylindrical body 14 and second cylindrical body 12 is selected from among a combination of Chromel and Alumel, combination of Chromel and copper, combination of Chromel and Constantan, combination of stainless steel grade 304 and Kovar, combination of Constantan and copper, and combination of copper and platinum-rhodium (in every combination, the former material is for the second cylindrical body 12 and the latter material is for the first cylindrical body 14).

Thicknesses of the first cylindrical body 14 and second cylindrical body 12 may be set independently of each other as appropriate, so as to satisfy strength determined according to the types of material used, service conditions of the probe, and the like. For example, the thicknesses can be set between about 0.05 mm and 0.30 mm. From the standpoint of safety and the like, preferably the second cylindrical body 12 is approximately two to three times as thick as the first cylindrical body 14.

In order for the first cylindrical body 14 and second cylindrical body 12 to run longitudinally by being electrically insulated from each other, the first cylindrical body 14 and second cylindrical body 12 may be spaced away from each other as illustrated or an insulating coating may be applied to an outer surface of the first cylindrical body 14 or inner surface of the second cylindrical body 12. Possible materials of such an insulating coating include organic insulating materials, glass, enamel, and glazes. A thread may be wound around the first cylindrical body 14 as a spacer. Needless to say, it is more desirable to apply the insulating coating 16 with the first cylindrical body 14 and second cylindrical body 12 spaced away from each other as shown in Figure 2.

The first cylindrical body 14 and second cylindrical body 12 are closed and joined at one end by the metal material of one of the first cylindrical body 14 and second cylindrical body 12 or another conducting material, forming a distal part 18 which electrically interconnects the first cylindrical body 14 and second cylindrical body 12. The distal part 18 also serves as a measuring junction of a thermocouple. The distal part 18 of the probe is not particularly limited in terms of shape, but hemispherical shapes are preferable from the viewpoint of manufacturability, temperature control, ease of insertion through the skin, and the like.

A minute communicating section 20 is formed near the distal part 18 to communicate between interior and exterior (an interior space of the first cylindrical body 14 and a space formed between the second cylindrical body 12 and first cylindrical body 14) of the first cylindrical body 14. The minute communicating section 20 may have any appropriate form such as a slit or hole formed in a cylindrical surface near the distal part of the first cylindrical body 14 as illustrated or a notch provided at the end of the first cylindrical body 14 forming a gap between the first cylindrical body 14 and distal part 18. The opening area of the minute communicating section 20 can be set as appropriate according to the necessary cooling capacity, type of refrigerant, flow rate and pressure of the circulated refrigerant, and other conditions, and may be, for example, in the range of about 1 × 10⁻³ mm² to 3 × 10⁻³ mm².

The cooling needle probe 10 is configured such that an appropriate medium (refrigerant) such as liquefied gas or compressed gas will be introduced into the first cylindrical body 14, passed through the minute communicating section 20, and lead out into the space between the second cylindrical body 12 and first cylindrical body 14 so as to evaporate (in the case of the liquefied gas) or adiabatically expand (in the case of the compressed gas) upon entry into the space, producing cold by the Joule-Thomson effect and thereby cooling the distal part 18. Alternatively, the medium may be introduced into the space between the second cylindrical body 12 and first cylindrical body 14 and subsequently the evaporated exhaust gas may be led out of the first cylindrical body 14.

The type of refrigerant used is not limited particularly, but Freon or an HFC (hydrofluorocarbon), for example, is preferable.

Temperature to which the distal part 18 is cooled is set as appropriate according to the body area to be operated on, purpose of the surgery, and the like, and may be a temperature close to a chilling temperature, i.e., a temperature of cold water, or a temperature below the chilling temperature, for example, a cryogenic temperature.

By applying the cooled distal part 18 of cooling needle probe 10 to a surgical target, cryogenic surgery or low-temperature surgery can be performed on the target site.

Being configured as described above, the cooling needle probe according to the first example of the present embodiment can be reduced to a very small diameter because there is no need to insert and use a temperature sensor for temperature measurement or temperature control in the probe. In particular, by reducing the probe diameter to less than 1 mm, for example, to about 0.5mm to 0.8mm, a minute region can be cooled accurately. Therefore, for example, by locally cooling the human brain rapidly using the cooling needle probe, epilepsy can be treated properly. In so doing, by freezing a local site, necrosing the local site, and returning the local site to body temperature in a cycle of, for example, a few tens of seconds, it is possible to treat only an epileptogenic zone (epileptic focus) accurately and to avoid the occurrence of residual disabilities.

Also, the cooling needle probe 10 according to the first example of the present embodiment can also be suitably used to take samples of cells frozen and deposited on the distal part 18 cooled below the chilling temperature. In this case, it is not strictly necessary to measure and control the temperature using a thermocouple or the like. Consequently, the above-described restrictions on the materials of the first cylindrical body 14 and second cylindrical body 12 of the probe do not apply in this case. Besides, the cells to be frozen and sampled are not limited to human cells, and may be cells of a living body other than human. Furthermore, the cells are not limited to those of living bodies.

Furthermore, the cooling needle probe 10 according to the first example of the present embodiment can also be used in biotechnology.

Also, with the cooling needle probe 10 according to the first example of the present embodiment, since a minute region is cooled using the distal part more minute than conventional ones and using a minute distal end of the second cylindrical body 12 as required, the minute region can be cooled reliably using smaller cooling capacity than conventional probes. Needless to say, it is desirable to use a refrigerant with a higher cold-generating capacity per unit quantity when measured under the same service conditions including temperature and pressure.

Next, a cooling needle probe according to a second example of the present embodiment will be described.

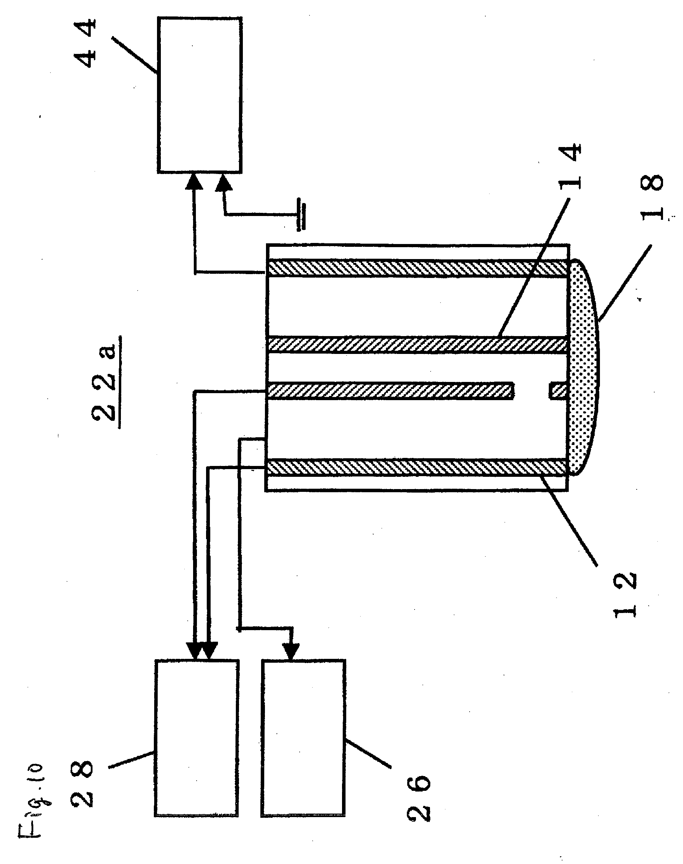

The cooling needle probe according to the second example of the present embodiment is similar to the cooling needle probe 10 according to the first example of the present embodiment except that the second cylindrical body 12 which runs longitudinally by being electrically insulated from the first cylindrical body 14 or the surroundings is connected to a biomedical signal measuring mechanism or an electrical stimulus supply mechanism (not shown) (see Figure 10 which shows and example in which the cylindrical body is connected to the biomedical signal measuring mechanism) so as to be able to measure biomedical signals or give electrical stimuli to a living body, via the distal part. This does not rule out the use of a switching mechanism in connecting the probe by switching between the biomedical signal measuring mechanism or electrical stimulus supply mechanism and a temperature-measuring device or the like which uses a thermocouple.

The biomedical signals widely include signals carrying physiological information resulting from activities of human beings or other living bodies (organisms), and are not limited particularly. However, biomedical signals especially useful in relation to cryogenic operations are brain wave signals and nerve signals.

This makes it possible to measure a brain wave signal via the distal part 18 applied to a surgical target using electric potential of an electrode (ground electrode) applied separately to any appropriate area as a reference potential, and thereby determine accurate position and character of, for example, an epileptogenic zone in a minute brain region. Also, by observing responses of the minute brain region to electrical stimuli, it is easy, for example, to confirm the minute region to be cooled or position the probe accurately in the minute region to be cooled. Also, based on whether or not epilepsy is induced by electrical stimuli, it is possible to determine whether or not the region is an epileptogenic zone (epileptic focus). Also, cooling procedures such as epilepsy treatment by cryogenic operation can be carried out effectively. That is, by taking results of brain wave measurements into consideration in the cryogenic operation, it is possible to necrose only an epileptic area as well as reduce surgical pain, postoperative scarring (alteration), postoperative complications, and surgical costs. This is also true for the other cooling needle probes described below.

Also, nerve signals can be measured during a cryogenic operation, for example, for necrosing a minute treated area of a nerve, allowing the cryogenic operation to be performed effectively. This is also true for the other cooling needle probes described below.

Since there is no need to provide a special electrode unit to measure the biomedical signal or give electrical stimuli to the living body, probe structure does not become complex and there is no obstruction to downsizing of the probe.

Regarding the electrode for reference potential, in the other examples described below, as with the present example, an electrode is installed separately from the probe to measure a reference potential by being applied to an appropriate area unless otherwise mentioned specifically.

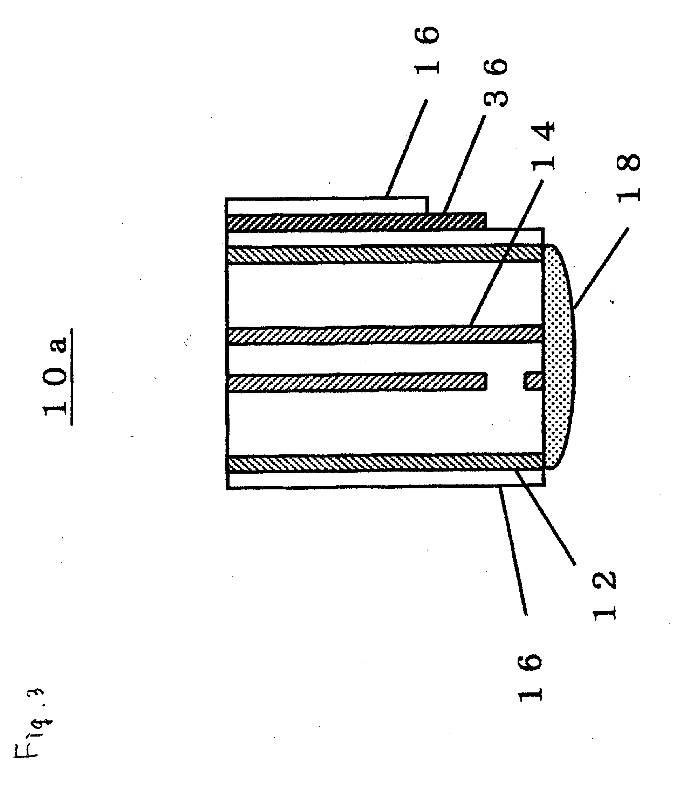

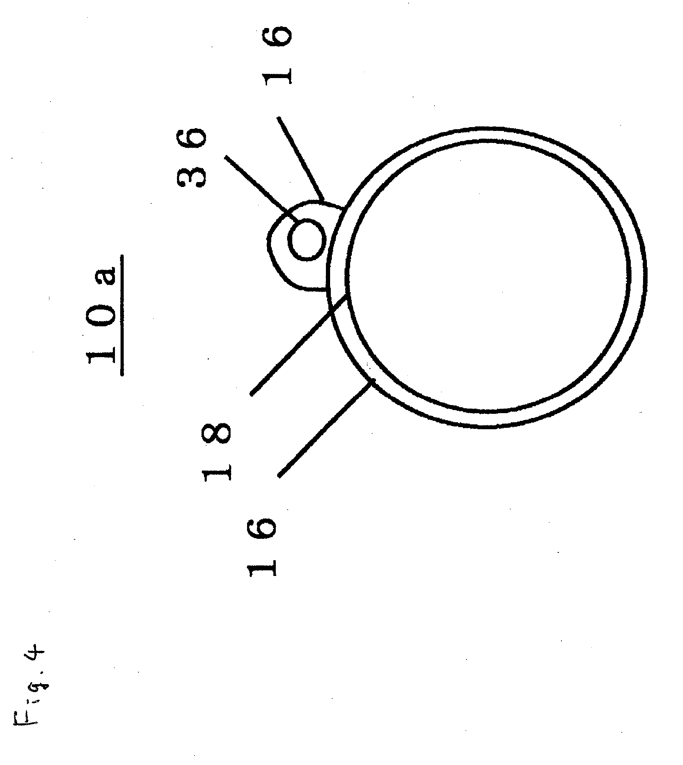

Next, a cooling needle probe according to a third example of the present embodiment will be described with reference to Figures 3 and 4.

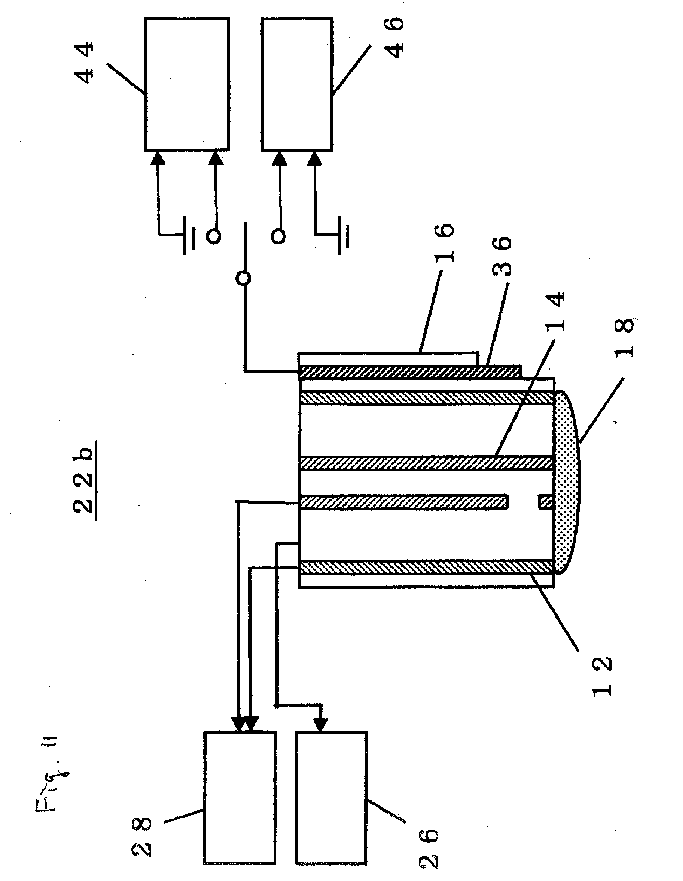

The cooling needle probe 10a according to the third example of the present embodiment, a schematic configuration of whose distal portion is shown as an example in Figures 3 and 4, has the same configuration as the cooling needle probe 10 according to the first example of the present embodiment except that the cooling needle probe 10a according to the third example of the present embodiment further comprises a first electrode unit 36 which runs longitudinally by being electrically insulated from surroundings and has a tip in or near the distal part 18, and that the first electrode unit 36 can measure biomedical signals and/or give electrical stimuli to a living body via the tip by being connected to a biomedical signal measuring mechanism or an electrical stimulus supply mechanism (not shown) or being connected to the biomedical signal measuring mechanism and the electrical stimulus supply mechanism (see Figure 11) in such a way as to be able to switch between the biomedical signal measuring mechanism and the electrical stimulus supply mechanism.

In the example shown in Figures 3 and 4, the cooling needle probe 10a is configured as follows: an external surface of the second cylindrical body 12 is covered with an insulating coating 16, the first electrode unit 36 formed into a rod-shaped lead is installed on one side of the insulating coating 16, and the first electrode unit 36 is further covered with an insulating coating 16. A tip of the first electrode unit 36 is exposed from the insulating coating 16 around it.

However, this is not restrictive, and the first electrode unit 36 may be installed at any appropriate location. For example, the first electrode unit 36 may be installed in a cavity between the first cylindrical body 14 and second cylindrical body 12 and the tip of the first electrode unit 36 may be exposed by penetrating the distal part 18 in an insulated state. Also, the first electrode unit 36 does not have its shape limited, and may be formed into any appropriate shape such as a strip.

In the cooling needle probe 10a, the second cylindrical body and the like can be used suitably as a ground connection terminal (ground electrode) for the biomedical signal measuring mechanism and the electrical stimulus supply mechanism.

Materials used for the first electrode unit 36 and insulating coating 16 are not limited particularly. For example, the same material as the first cylindrical body 14 and second cylindrical body 12 of the cooling needle probe 10 according to the first example of the present embodiment may be used for the first electrode unit 36 and the same material as the insulating coating of the cooling needle probe 10 according to the first example of the present embodiment may be used for the insulating coating 16. However, from the standpoint of corrosion prevention, platinum or gold is preferable. This is also true for the electrode units and the like according to the other examples described below.

Description will be given of an operation method and operation of the cooling needle probe 10a according to the third example of the present embodiment configured as described above.

With the distal part 18 of the cooling needle probe 10a applied, for example, to a surgical target in the human brain, electrical stimuli are given via the first electrode unit 36. For example, by applying pulsed current or voltage, it is possible to observe responses of a minute brain region to electrical stimuli, making it easy, for example, to confirm the minute region to be cooled or position the probe accurately in the minute region to be cooled. Also, based on whether or not epilepsy is induced by electrical stimuli, it is possible to determine whether or not the region is an epileptogenic zone (epileptic focus).

On the other hand, with the distal part 18 of the cooling needle probe 10a applied, for example, to a surgical target in the human brain, by measuring brain waves in a minute region of the human brain to be cooled accurately, it is possible to determine accurate position and character of, for example, an epileptogenic zone in the minute region. This makes it possible to carry out epilepsy treatment by cryogenic operation more effectively.

Also, by giving an electrical stimulus and taking a brain wave measurement alternately and thereby measuring brain waves produced in response to the electrical stimuli to a minute region of the human brain to be cooled accurately, it is possible to determine accurate position and character of, for example, an epileptogenic zone in the minute region more properly. This makes it possible to carry out epilepsy treatment by cryogenic operation still more effectively.

Next, a variation of the cooling needle probe according to the third example of the present embodiment will be described.

The cooling needle probe according to the variation has the same configuration as the cooling needle probe 10a according to the third example of the present embodiment except that one of (1) the first electrode unit 36 and (2) the second cylindrical body 12 which runs longitudinally by being electrically insulated from the first cylindrical body 14 or the surroundings can measure biomedical signals from a predetermined area of a living body (e.g., a surgical target in the human brain) and/or give electrical stimuli to the predetermined area of the living body, via the distal part 18 or the tip, by being connected to the biomedical signal measuring mechanism or electrical stimulus supply mechanism or being connected to the biomedical signal measuring mechanism and electrical stimulus supply mechanism in such a way as to be able to switch between the biomedical signal measuring mechanism and electrical stimulus supply mechanism and that the other of the two can measure biomedical signals from an area other than the predetermined area by being connected to the biomedical signal measuring mechanism.

This is suitable because the reference potential can be measured near the predetermined area using the probe without installing a separate electrode for use to measure the reference potential. Incidentally, an electrode for use to measure the reference potential may be installed separately while using the probe to measure biomedical signals from the predetermined area and an area very close to the predetermined area.

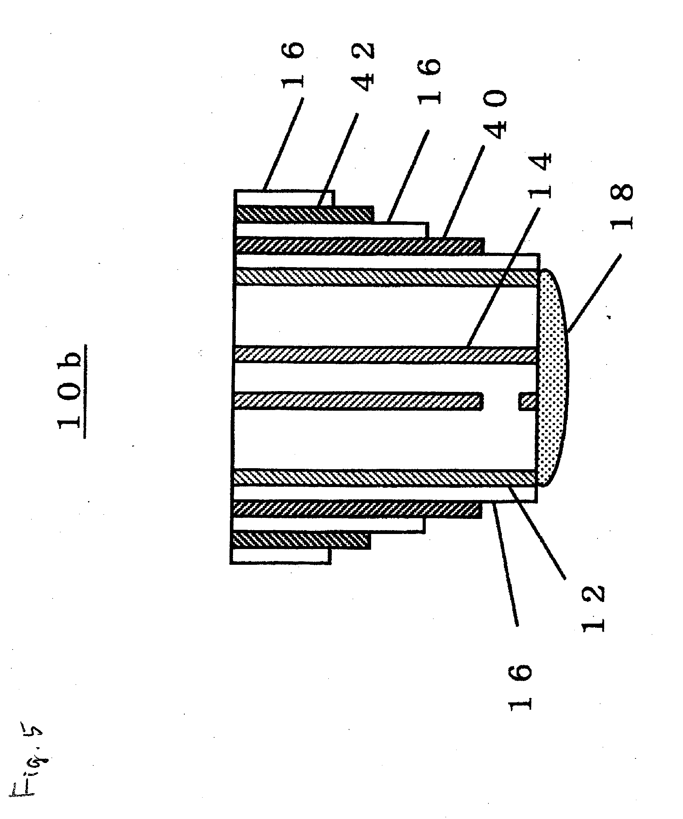

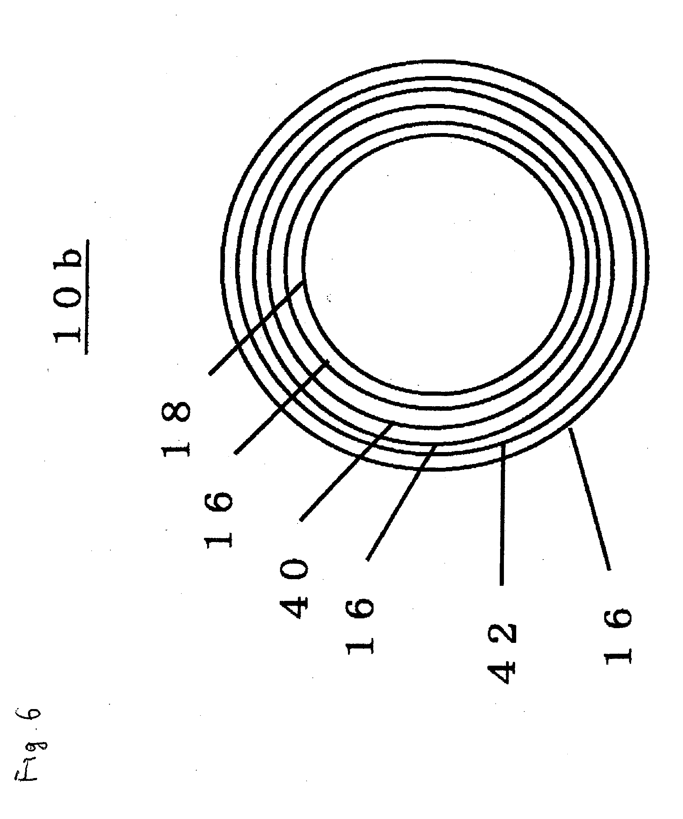

Next, a cooling needle probe according to a fourth example of the present embodiment will be described with reference to Figures 5 and 6.

The cooling needle probe 10b according to the fourth example of the present embodiment, a schematic configuration of whose distal portion is shown as an example in Figures 5 and 6, has the same configuration as the cooling needle probe 10 according to the first example of the present embodiment except that the cooling needle probe 10b further includes a second electrode unit (dielectric electrode) 42 and third electrode unit 40 each of which runs longitudinally by being electrically insulated from surroundings and has a tip in or near the distal part 18, that the second electrode unit 42 can measure biomedical signals via the tip by being connected to a biomedical signal measuring mechanism and that the third electrode unit 40 can give electrical stimuli to a living body via the tip by being connected to an electrical stimulus supply mechanism.

Specifically, the cooling needle probe 10b differs from the cooling needle probe 10a according to the third example of the present embodiment in that the second electrode unit 42 has a tubular shape and that the cooling needle probe 10b is equipped with the third electrode unit 40 tubular in shape. An external surface of the third electrode unit 40 is covered with an insulating coating 16, the second electrode unit 42 is installed on an external surface of the insulating coating 16, and an external surface of the second electrode unit 42 is covered with an insulating coating 16. A tip of the second electrode unit 42 and tip of the third electrode unit 40 are exposed from the respective insulating coatings 16.

However, this is not restrictive, and the second electrode unit 42 and third electrode unit 40 may be installed at any appropriate locations. For example, positions of the second electrode unit 42 and third electrode unit 40 may be exchanged.

Description will be given of an operation method and operation of the cooling needle probe 10b configured as described above.

With the distal part 18 of the cooling needle probe 10b applied, for example, to a surgical target in the human brain, electrical stimuli are applied to the surgical target via the third electrode unit 40. Next, brain waves are measured at the surgical target via the second electrode unit 42. By analyzing and evaluating patterns of the brain waves, it is possible to determine accurate position and character of, for example, an epileptogenic zone in the minute region. This makes it possible to carry out epilepsy treatment by cryogenic operation more effectively. If nerve signals are measured and analyzed by applying electrical stimuli to a nerve instead of the brain as a surgical target, nerve treatment by cryogenic operation can be carried out more effectively.

Regardless of the above configuration, both second electrode unit and third electrode unit may be first used to apply electrical stimuli, and then switched to measuring brain waves. Biomedical signals may be measured via the second electrode unit and third electrode unit using a measuring amplifier, whose ground may be connected to the second cylindrical body or to an electrode installed in the living body quite separately from the probe.

In so doing, it will be more suitable to use a self-organizing relationship network technique developed by the present inventors. The self-organizing relationship network is a neural network obtained by adding a function to express input-output relationship to a self-organizing map proposed by Teuvo Kohonen. The self-organizing relationship network is a simulation model of a brain mapping function, and the technique performs modeling using input-output pairs obtained by trial and error and their evaluation values.

Next, a first variation of the cooling needle probe according to the fourth example of the present embodiment will be described.

The cooling needle probe according to the first variation has the same configuration as the cooling needle probe 10b according to the fourth example of the present embodiment except that one of the (1) the second cylindrical body 42, (2) the third electrode unit 40, and (3) the second cylindrical body 12 which runs longitudinally by being electrically insulated from the first cylindrical body 14 or the surroundings can measure biomedical signals from a predetermined area of a living body via a tip by being connected to a biomedical signal measuring mechanism, another of the three can give electrical stimuli to the predetermined area of the living body via the tip by being connected to an electrical stimulus supply mechanism, and the remaining one can measure biomedical signals from an area other than the predetermined area.

This is suitable because the reference potential can be measured near the predetermined area using the probe without installing a separate electrode for use to measure the reference potential.

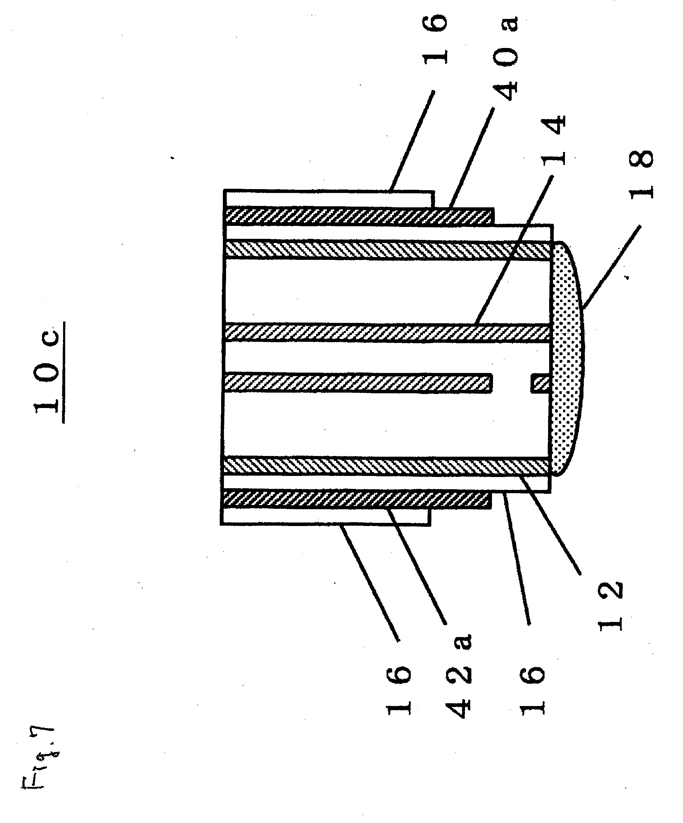

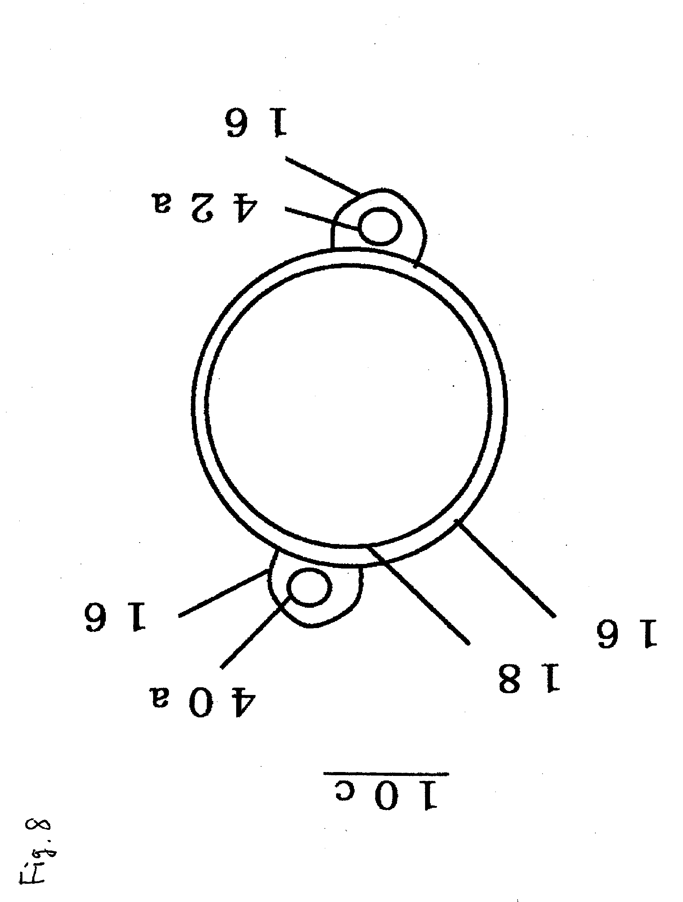

Next, a second variation of the cooling needle probe 10b according to the fourth example of the present embodiment will be described with reference to Figures 7 and 8.

The cooling needle probe 10c according to the second variation, a schematic configuration of whose distal portion is shown as an example in Figures 7 and 8, differs from the cooling needle probe 10b in that a third electrode unit 40a and second electrode unit 42a each formed into a rod-shaped lead are installed on diametrically opposite sides of the outer surface of the insulating coating 16 which covers the second cylindrical body 12.

This is preferable because of reduced stray capacitance compared to when the second electrode unit 42a and third electrode unit 40a are formed concentrically as in the case of the second electrode unit 42 and third electrode unit 40 or when the first electrode unit 36 and second cylindrical body 12 tubular in shape are used.

Incidentally, the second electrode unit 42a and third electrode unit 40a do not have their shape limited, and may be formed into a strip-shaped lead or any other appropriate shape.

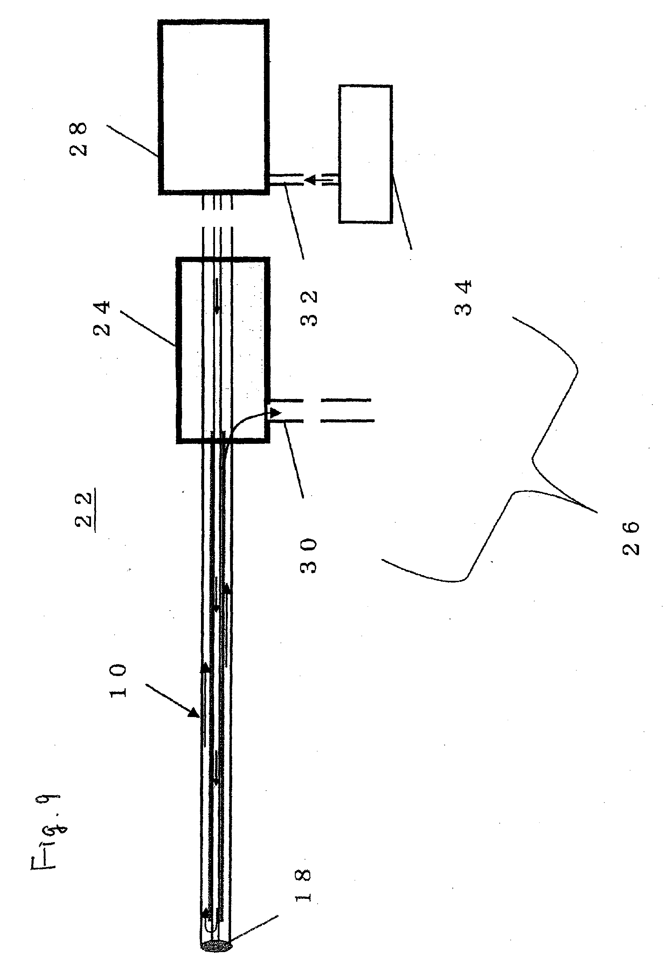

Next, a cooling system according to a fifth example of the present embodiment will be described with reference to Figure 9.

The cooling system 22 according to the fifth example of the present embodiment, whose schematic configuration is shown in Figure 9 as an example, includes the cooling needle probe 10 according to the first example of the present embodiment, a probe grip 24 installed on the side opposite the distal part 18 of the cooling needle probe 10, a refrigerant supply and discharge mechanism 26, and a refrigerant quantity control mechanism 28.

The probe grip 24 is used to grip and operate the cooling needle probe 10 and is connected with a discharge pipe 30 and supply pipe 32 of the refrigerant supply and discharge mechanism 26 described later.

The refrigerant supply and discharge mechanism 26 is used to supply a refrigerant (cooling medium) to the cooling needle probe 10 and discharge evaporated exhaust refrigerant gas to an appropriate place where it is safe to discharge the exhaust gas from the cooling needle probe 10.

The refrigerant supply and discharge mechanism 26 includes a storage unit 34 used to store the refrigerant, the supply pipe 32 used to supply the refrigerant from the storage unit 34 to the cooling needle probe 10 by connecting the storage unit 34 to the cooling needle probe 10, and the discharge pipe 30 used to discharge the exhaust refrigerant gas evaporated in the cooling needle probe 10 from the cooling needle probe 10. Preferably the discharge pipe 30 and supply pipe 32 are long enough not to interfere with operation of the cooling needle probe 10 and have flexibility. However, when the exhaust gas is very small in amount, the discharge pipe 30 may be short or may be practically omitted.

In this case, the refrigerant may be supplied under pressure to the cooling needle probe 10 using pressure of the refrigerant or using a pressure source such as a pump (not shown), as required. On the other hand, the exhaust gas may be released into the air by residual pressure or sucked using an appropriate suction source such as a pump (not shown) together with or without a compression source.

The refrigerant quantity control mechanism 28 is equipped with a thermocouple reference junction (not shown) connected with respective ends of the first cylindrical body 14 and second cylindrical body 12. Also, the refrigerant quantity control mechanism 28 includes a temperature display and control unit (not shown) which displays temperature of the distal part 18 of the cooling needle probe 10 as required and keeps the distal part 18 of the cooling needle probe 10 at a predetermined temperature by opening and closing a supply solenoid valve of the supply pipe 32 and a discharge solenoid valve of the discharge pipe 30 (neither is shown), based on a signal resulting from an electromotive force generated between the reference junction and measuring junction.

To illustrate by example how to use the cooling system 22, first the distal part 18 of the cooling probe 10 is pushed in, for example, through the scalp by gripping the probe grip 24. When the distal part of the probe reaches the surgical target, the refrigerant is supplied to the cooling needle probe 10 and circulation of the refrigerant is stabilized by operating the refrigerant quantity control mechanism 28 in manual mode or by temporarily setting a proper temperature with the temperature display and control unit in automatic mode. Consequently, by cold transmitted from the distal part 18 to the interior of the brain cooled, for example, to a few degrees centigrade, the interior of the brain is locally cooled rapidly below room temperature, for example, to about 20°C.

Since an amount of cold removed from the distal part 18 of the cooling needle probe 10 by the surgical site is smaller than in the case of conventional probes, an ample amount of cold sufficiently larger than the removed amount of cold can be supplied. Also, since the distal part 18 of the probe 10 which is a cold source coincides with the measuring junction of the thermocouple, temperature changes can be controlled to within a fluctuation range of 1°C or less, making it possible to perform accurate surgery.

Next, a cooling system according to a sixth example of the present embodiment will be described with reference to Figure 10.

The cooling system 22a according to the sixth example of the present embodiment, whose configuration is schematically shown in Figure 10 uses the cooling needle probe according to the second example of the present embodiment. The cooling system 22a not only includes a probe grip (not shown), refrigerant supply and discharge mechanism 26, and refrigerant quantity control mechanism 28 as with the cooling system 22 according to the fourth example of the present embodiment, but also includes a biomedical signal measuring mechanism or electrical stimulus supply mechanism connected to the first cylindrical body 14 or second cylindrical body 12. Figure 10 shows an example of how the biomedical signal measuring mechanism 44 is connected to the second cylindrical body 12. In this case, the biomedical signal measuring mechanism 44 is equipped with a signal amplifier which amplifies biomedical signals and a biomedical signal display and recorder.

By measuring biomedical signals or giving electrical stimuli to the living body before or after a cooling operation, it is possible to take good advantage of the cooling needle probe according to the second example of the present embodiment.

Next, a cooling system according to a seventh example of the present embodiment will be described with reference to Figure 11.

The cooling system 22b according to the seventh example of the present embodiment, whose configuration is schematically shown in Figure 11 uses the cooling needle probe 10a according to the third example of the present embodiment. The cooling system 22a includes a probe grip (not shown), refrigerant supply and discharge mechanism 26, and refrigerant quantity control mechanism 28 as with the cooling system 22 according to the fourth example of the present embodiment. Besides, the first electrode unit 36 is equipped with a biomedical signal measuring mechanism 44 and/or electrical stimulus supply mechanism 46.

If, for example, an electrical stimulus is given to the brain and then a brain wave measurement is taken by switching between the biomedical signal measuring mechanism 44 and electrical stimulus supply mechanism 46, as appropriate, before or after a cooling operation, it is possible to take good advantage of the cooling needle probe 10a according to the third example of the present embodiment. When, for example, current stimuli are given using the electrical stimulus supply mechanism 46, if a pulsed current with a pulse amplitude of 50 µA, pulse width of 200 µsec, and repetition frequency of 50 Hz is applied to the hippocampus of a rat for a few seconds, generation of epilepsy waves can be observed.

As a variation, the cooling system 22b according to the seventh example of the present embodiment may be configured such that using the cooling needle probe according to the variation of the third example of the present embodiment, one of (1) the second cylindrical body 12 which runs longitudinally by being electrically insulated from the first cylindrical body 14 or the surroundings and (2) the first electrode unit 36 can measure biomedical signals from a predetermined area of a living body and/or give electrical stimuli to the predetermined area of the living body, via the distal part 18 or the tip, by being connected to the biomedical signal measuring mechanism 44 or electrical stimulus supply mechanism 46 or being connected to the biomedical signal measuring mechanism 44 and electrical stimulus supply mechanism 46 in such a way as to be able to switch between the biomedical signal measuring mechanism 44 and electrical stimulus supply mechanism 44 and that the other of the two can measure biomedical signals (including a reference potential, in this case) from an area other than the predetermined area by being connected to the biomedical signal measuring mechanism 44.

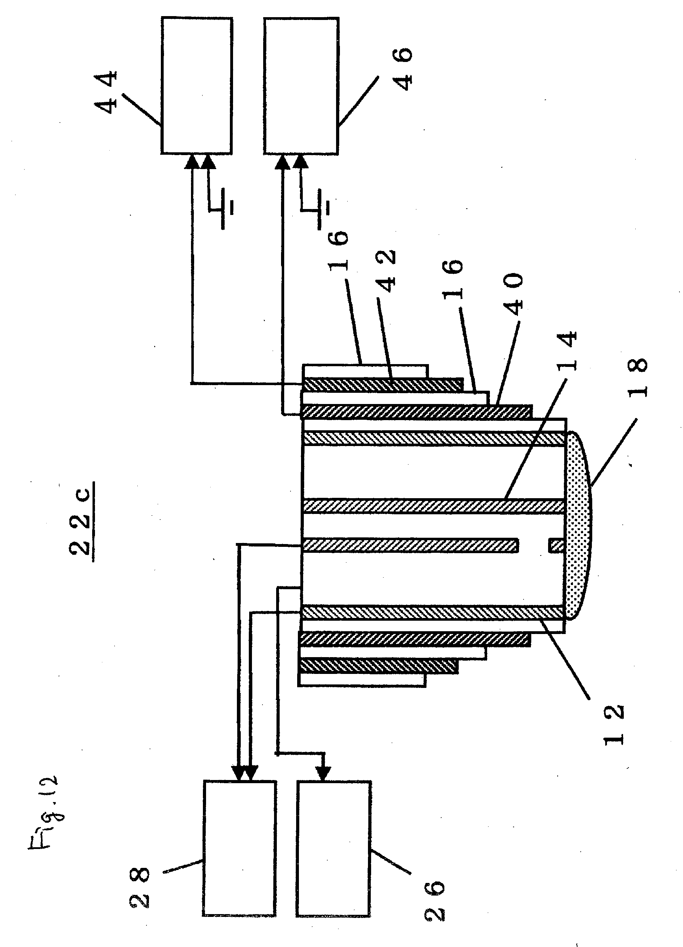

Next, a cooling system according to an eighth example of the present embodiment will be described with reference to Figure 12.

The cooling system 22c according to the eighth example of the present embodiment, whose configuration is schematically shown in Figure 12 uses the cooling needle probe 10b according to the fourth example of the present embodiment. The cooling system 22c not only includes a probe grip (not shown), refrigerant supply and discharge mechanism 26, and refrigerant quantity control mechanism 28 as with the cooling system 22 according to the fourth example of the present embodiment, but also includes the biomedical signal measuring mechanism 44 and electrical stimulus supply mechanism 46 connected, respectively, with the second electrode unit 42 and third electrode unit 40.

If, for example, an electrical stimulus is given to the brain and then a brain wave measurement is taken using the biomedical signal measuring mechanism 44 and electrical stimulus supply mechanism 46, as appropriate, before or after a cooling operation, it is possible to take good advantage of the cooling needle probe 10b according to the fourth example of the present embodiment.

As a variation, the cooling system 22c according to the eighth example of the present embodiment may be configured such that using the first variation of the cooling needle probe according to the fourth example of the present embodiment, one of (1) the second electrode unit 42, (2) the third electrode unit 40, and (3) the second cylindrical body 12 which runs longitudinally by being electrically insulated from the first cylindrical body 14 or the surroundings can measure biomedical signals from a predetermined area of a living body via a tip by being connected to the biomedical signal measuring mechanism 44, another of the three can give electrical stimuli to the predetermined area of the living body via the tip by being connected to the electrical stimulus supply mechanism 46, and the remaining one can measure biomedical signals (including a reference potential, in this case) from an area other than the predetermined area.

## Claims

1. A cooling system (22) comprising:
a cooling needle probe (10, 10a) comprising a first cylindrical body (14) and a second cylindrical body (12) which are made of thin-walled dissimilar metals and run longitudinally by being electrically insulated from each other, where the second cylindrical body (12) is installed around the first cylindrical body (14), wherein the first cylindrical body (14) and the second cylindrical body (12) are closed and joined at one end, forming a distal part (18) which electrically interconnects the first cylindrical body (14) and the second cylindrical body (12); a minute communicating section (20) being formed near the distal part (18) to communicate between an interior and an exterior of the first cylindrical body (14);
wherein
the cooling needle probe (10, 10a) is configured such that a refrigerant flowing into one of: a cavity between the second cylindrical body (12) and the first cylindrical body (14); and a cavity in the first cylindrical body (14), evaporates or adiabatically expands upon passage through the minute communicating section (20), thereby generating cold, which flows into the other cavity to cool the distal part (18); and
the distal part (18) serves as a measuring junction of a thermocouple while the first cylindrical body (14) serves as a compensating lead of the thermocouple,
the cooling system (22) further comprising:
a probe grip (10) installed on a side opposite the distal part (18) of the cooling needle probe (10, 10a); a refrigerant supply and discharge mechanism (26) adapted to supply a refrigerant to the cooling needle probe (10, 10a) and discharge the refrigerant after cold is released; and a refrigerant quantity control mechanism (28) adapted to control a supply quantity of the refrigerant based on a signal from the thermocouple, **characterized in that** the second cylindrical body (12) serves as a further compensating lead of the thermocouple and
the refrigerant quantity control mechanism (28) is equipped with a thermocouple reference junction connected with respective ends of the first cylindrical body and second cylindrical body.

2. The cooling system (22) according to claim 1, **characterized in that** a combination of materials for the first cylindrical body (14) and the second cylindrical body (12) is selected from among a combination of Chromel and Alumel, a combination of Chromel and copper, a combination of Chromel and Constantan, a combination of stainless steel grade 304 and Kovar, a combination of Constantan and copper, and a combination of platinum and platinum-rhodium.

3. The cooling system (22) according to claim 1, **characterized in that** the probe is 1 mm or less in diameter.

4. The cooling system (22) according to claim 1, **characterized in that** the second cylindrical body (12) which runs longitudinally by being electrically insulated from the first cylindrical body (14) or surroundings is connected to a biomedical signal measuring mechanism or an electrical stimulus supply mechanism so as to be able to measure a biomedical signal or give an electrical stimulus to a living body, via the distal part (18).

5. The cooling system (22) according to claim 1, wherein the cooling needle probe (10, 10a) further comprises a first electrode unit (36) which runs longitudinally by being electrically insulated from surroundings and has a tip in or near the distal part (18), **characterized in that** the first electrode unit (36) can measure a biomedical signal via the tip and/or give an electrical stimulus to a living body via the tip by being connected to a biomedical signal measuring mechanism or an electrical stimulus supply mechanism or being connected to the biomedical signal measuring mechanism and the electrical stimulus supply mechanism in such a way as to be able to switch between the biomedical signal measuring mechanism and the electrical stimulus supply mechanism.

6. The cooling system (22) according to claim 1, wherein the cooling needle probe (10, 10a) further comprises a first electrode unit (36) which runs longitudinally by being electrically insulated from surroundings and has a tip in or near the distal part (18), **characterized in that** one of the second cylindrical body (12) which runs longitudinally by being electrically insulated from the first cylindrical body (14) or surroundings and the first electrode unit (36) can measure a biomedical signal from a predetermined area of a living body and/or give an electrical stimulus to the predetermined area of the living body, via the distal part (18) or the tip, by being connected to a biomedical signal measuring mechanism or an electrical stimulus supply mechanism or being connected to the biomedical signal measuring mechanism and the electrical stimulus supply mechanism in such a way as to be able to switch between the biomedical signal measuring mechanism and the electrical stimulus supply mechanism, and the other of the two can measure a biomedical signal from an area other than the predetermined area by being connected to the biomedical signal measuring mechanism.

7. The cooling system (22) according to claim 1, wherein the cooling needle probe (10, 10a) further comprises a second electrode unit (42, 42a) and a third electrode unit (40, 40a) each of which runs longitudinally by being electrically insulated from surroundings and has a tip in or near the distal part (18), **characterized in that** the second electrode unit (42, 42a) can measure a biomedical signal via the tip by being connected to a biomedical signal measuring mechanism; and the third electrode unit (40, 40a) can give an electrical stimulus to a living body via the tip by being connected to an electrical stimulus supply mechanism.

8. The cooling system (22) according to claim 1, wherein the cooling needle probe (10, 10a) further comprises a second electrode unit (42, 42a) and a third electrode unit (40, 40a) each of which runs longitudinally by being electrically insulated from surroundings and has a tip in or near the distal part (18), **characterized in that** one of the second electrode unit (42, 42a), the third electrode unit (40, 40a), and the second cylindrical body (12) can measure a biomedical signal from a predetermined area of a living body via the tip by being connected to a biomedical signal measuring mechanism, where the second cylindrical body (12) runs longitudinally by being electrically insulated from the first cylindrical body (14) or surroundings; another of the three can give an electrical stimulus to the predetermined area of the living body via the tip by being connected to an electrical stimulus supply mechanism; and the remaining one can measure a biomedical signal from an area other than the predetermined area.

9. The cooling system (22) according to claim 4; wherein the cooling system further comprises a biomedical signal measuring mechanism or an electrical stimulus supply mechanism connected with the first cylindrical body (14) or the second cylindrical body (12).

10. The cooling system (22) according to claim 5; wherein the cooling system further comprises a biomedical signal measuring mechanism and/or an electrical stimulus supply mechanism connected with the first electrode unit (36).

11. The cooling system (22) according to claim 6; wherein the cooling system further comprises a biomedical signal measuring mechanism and/or an electrical stimulus supply mechanism connected with one of the second cylindrical body (12) and the first electrode unit (36), where the second cylindrical body (12) runs longitudinally by being electrically insulated from the first cylindrical body (14) or surroundings.

12. The cooling system (22) according to claim 7; wherein the cooling system further comprises a biomedical signal measuring mechanism connected with the second electrode unit (42, 42a); and an electrical stimulus supply mechanism connected with a third electrode unit (40, 40a).

13. The cooling system (22) according to claim 8; wherein the cooling system further comprises a biomedical signal measuring mechanism and an electrical stimulus supply mechanism connected with one of the second electrode unit (42, 42a), a third electrode unit (40, 40a), and the second cylindrical body (12), where the second cylindrical body (12) runs longitudinally by being electrically insulated from the first cylindrical body (14) or surroundings.

## Patentansprüche

1. Kühlsystem (22) umfassend:
eine Kühlnadelsonde (10, 10a), umfassend einen ersten zylindrischen Körper (14) und einen zweiten zylindrischen Körper (12), die aus dünnwandigen ungleichen Metallen hergestellt sind und elektrisch voneinander isoliert in Längsrichtung verlaufen, wobei der zweite zylindrische Körper (12) um den ersten zylindrischen Körper (14) verläuft, und der erste zylindrische Körper (14) und der zweite zylindrische Körper (12) an einem Ende geschlossen und miteinander verbunden sind, so dass ein distaler Teil (18) erhalten wird, der den ersten zylindrischen Körper (14) und den zweiten zylindrischen Körper (12) elektrisch verbindet, wobei ein winziger kommunizierender Abschnitt (20) nahe dem distalen Teil (18) gebildet wird, der zwischen der Innenseite und der Außenseite des zylindrischen Körpers (14) kommuniziert;
wobei
die Kühlnadelsonde (10, 10a) derart konfiguriert ist, dass ein Kühlmittel, das in eine der folgenden Komponenten, d.h. in einen Hohlraum zwischen dem zweiten zylindrischen Körper (12) und dem ersten zylindrischen Körper (14) und einen Hohlraum im ersten zylindrischen Körper (14), strömt, beim Durchtritt durch den winzigen kommunizierenden Abschnitt (20) verdampft oder sich adiabatisch ausdehnt, wodurch Kälte erzeugt wird, die in den anderen Hohlraum übertritt und den distalen Teil (18) kühlt; und
der distale Teil (18) als Messverbindung zu einem Thermoelement dient, wohingegen der erste zylindrische Körper (14) als Ausgleichsleitung für das Thermoelement dient,
wobei das Kühlsystem (22) zudem umfasst:
eine Sondenhalterung (10), die auf einer Seite gegenüber dem distalen Teil (18) der Kühlnadelsonde (10, 10a) angebracht ist;
einen Kühlmittelzufuhr- und Auslassmechanismus (26), der ein Kühlmittel zur Kühlnadelsonde (10, 10a) leitet und das Kühlmittel auslässt, sobald Kälte freigesetzt wurde; und
einen Kühlmittelmengen-Steuermechanismus (28), mit dem die Menge des zuzuführenden Kühlmittels auf der Basis eines Signals vom Thermoelement gesteuert wird;
**dadurch gekennzeichnet, dass** der zweite zylindrische Körper (12) als weitere Ausgleichsleitung des Thermoelementes dient, und
der Mechanismus zum Steuern der Kühlmittelmenge (28) eine Thermoelement-Referenz-Verbindung aufweist, die mit den jeweiligen Enden des ersten und zweiten zylindrischen Körpers verbunden sind.

2. Kühlsystem (22) nach Anspruch 1, **dadurch gekennzeichnet, dass** eine Kombination von Materialien für den ersten zylindrischen Körper (14) und den zweiten zylindrischen Körper (12) ausgewählt ist aus einer Kombination von Chromel und Alumel, einer Kombination von Chromel und Kupfer, einer Kombination von Chromel und Konstantan, einer Kombination von Edelstahl Grad 304 und Kovar, einer Kombination von Konstantan und Kupfer und einer Kombination von Platin und Platin-Rhodium.

3. Kühlsystem (22) nach Anspruch 1, **dadurch gekennzeichnet, dass** der Durchmesser der Sonde 1 mm oder kleiner ist.

4. Kühlsystem (22) nach Anspruch 1, **dadurch gekennzeichnet, dass** der zweite zylindrische Körper (12), der von dem ersten zylindrischen Körper (14) oder der Umgebung elektrisch isoliert in Längsrichtung verläuft, mit einem Mechanismus zum Messen eines biomedizinischen Signals oder einem Mechanismus zum Übermitteln eines elektrischen Reizes verbunden ist, so dass man über den distalen Teil (18) ein biomedizinisches Signal messen oder einen elektrischen Reiz an einen lebenden Körper übermitteln kann.

5. Kühlsystem (22) nach Anspruch 1, wobei die Kühlnadelsonde (10, 10a) zudem eine erste Elektrodeneinheit (36) umfasst, die von der Umgebung elektrisch isoliert in Längsrichtung verläuft, und eine Spitze in oder nahe dem distalen Teil (18) aufweist, **dadurch gekennzeichnet, dass** die erste Elektrodeneinheit (36) über die Spitze ein biomedizinisches Signal messen kann und/oder über die Spitze einen elektrischen Reiz an einen lebenden Körper übermitteln kann, indem sie mit einem Mechanismus zum Messen des biomedizinischen Signals oder einem Mechanismus zum Übermitteln eines elektrischen Reizes verbunden ist, oder indem sie derart mit dem Mechanismus zum Messen des biomedizinischen Signals und dem Mechanismus zum Übermitteln eines elektrischen Reizes verbunden ist, dass man zwischen dem Mechanismus zum Messen des biomedizinischen Signals und dem Mechanismus zum Übermitteln eines elektrischen Reizes umschalten kann.

6. Kühlsystem (22) nach Anspruch 1, wobei die Kühlnadelsonde (10, 10a) zudem eine erste Elektrodeneinheit (36) umfasst, die von der Umgebung elektrisch isoliert in Längsrichtung verläuft, und eine Spitze in oder nahe dem distalen Teil (18) aufweist, **dadurch gekennzeichnet, dass** einer von dem zweiten zylindrischen Körper (12), der vom ersten zylindrischen Körper (14) oder der Umgebung elektrisch isoliert in Längsrichtung verläuft, und der ersten Elektrodeneinheit (36) über den distalen Teil (18) oder die Spitze ein biomedizinisches Signal von einem festgelegten Bereich eines lebenden Körpers messen kann und/oder einen elektrischen Reiz an den festgelegten Bereich des lebenden Körpers übermitteln kann, indem er bzw. sie mit dem Mechanismus zum Messen des biomedizinischen Signals oder dem Mechanismus zum Übermitteln eines elektrischen Reizes verbunden ist, oder derart mit dem Mechanismus zum Messen des biomedizinischen Signals und dem Mechanismus zum Übermitteln eines elektrischen Reizes verbunden ist, dass man zwischen dem Mechanismus zum Messen des biomedizinischen Signals und dem Mechanismus zum Übermitteln eines elektrischen Reizes umschalten kann, und der jeweils andere der beiden Mechanismen ein biomedizinisches Signal aus einem anderen Bereich als dem festgelegten Bereich messen kann, indem er mit dem Mechanismus zum Messen des biomedizinischen Signals verbunden ist.

7. Kühlsystem (22) nach Anspruch 1, wobei die Kühlnadelsonde (10, 10a) zudem eine zweite Elektrodeneinheit (42, 42a) und eine dritte Elektrodeneinheit (40, 40a) umfasst, die jeweils von der Umgebung elektrisch isoliert in Längsrichtung verlaufen, und sie eine Spitze in oder nahe dem distalen Teil (18) aufweist, **dadurch gekennzeichnet, dass** die zweite Elektrodeneinheit (42, 42a) über die Spitze ein biomedizinisches Signal messen kann, indem sie mit einem Mechanismus zum Messen des biomedizinischen Signals verbunden ist; und die dritte Elektrodeneinheit (40, 40a) über die Spitze einen elektrischen Reiz an einen lebenden Körper übermitteln kann, indem sie mit einem Mechanismus zum Übermitteln eines elektrischen Reizes verbunden ist.

8. Kühlsystem (22) nach Anspruch 1, wobei die Kühlnadelsonde (10, 10a) zudem eine zweite Elektrodeneinheit (42, 42a) und eine dritte Elektrodeneinheit (40, 40a) umfasst, die jeweils von der Umgebung elektrisch isoliert in Längsrichtung verlaufen, und sie eine Spitze in oder nahe dem distalen Teil (18) aufweist, **dadurch gekennzeichnet, dass** eine von der zweiten Elektrodeneinheit (42, 42a), der dritten Elektrodeneinheit (40, 40a) und des zweiten zylindrischen Körpers (12) über die Spitze ein biomedizinisches Signal aus einem festgelegten Bereich eines lebenden Körpers messen kann, indem sie bzw. er mit einem Mechanismus zum Messen des biomedizinischen Signals verbunden ist, wobei der zweite zylindrische Körper (12) von dem ersten zylindrischen Körper (14) oder der Umgebung elektrisch isoliert in Längsrichtung verläuft, wobei ein weiterer der drei über die Spitze einen elektrischen Reiz an den festgelegten Bereich des lebenden Körpers übermitteln kann, indem sie bzw. er mit einem Mechanismus zum Übermitteln des elektrischen Reizes verbunden ist; und der verbleibende ein biomedizinisches Signal aus einem anderen Bereich als den festgelegten Bereich messen kann.

9. Kühlsystem (22) nach Anspruch 4, wobei das Kühlsystem zudem einen Mechanismus zum Messen eines biomedizinischen Signals oder einen Mechanismus zum Übermitteln eines elektrischen Reizes umfasst, der mit dem ersten zylindrischen Körper (14) oder dem zweiten zylindrischen Körper (12) verbunden ist.

10. Kühlsystem (22) nach Anspruch 5, wobei das Kühlsystem zudem einen Mechanismus zum Messen eines biomedizinischen Signals und/oder einen Mechanismus zum Übermitteln eines elektrischen Reizes umfasst, der mit der ersten Elektrodeneinheit (36) verbunden ist.

11. Kühlsystem (22) nach Anspruch 6, wobei das Kühlsystem zudem einen Mechanismus zum Messen eines biomedizinischen Signals und/oder einen Mechanismus zum Übermitteln eines elektrischen Reizes umfasst, der mit einem von dem zweitem zylindrischen Körper (12) und der ersten Elektrodeneinheit (36) verbunden ist, wobei der zweite zylindrische Körper (12) von dem ersten zylindrischen Körper (14) oder der Umgebung elektrisch isoliert in Längsrichtung verläuft.

12. Kühlsystem (22) nach Anspruch 7, wobei das Kühlsystem zudem einen Mechanismus zum Messen eines biomedizinischen Signals, der mit der zweiten Elektrodeneinheit (42, 42a) verbunden ist, und einen Mechanismus zum Übermitteln eines elektrischen Reizes, der mit einer dritten Elektrodeneinheit (40, 40a) verbunden ist, umfasst.

13. Kühlsystem (22) nach Anspruch 8, wobei das Kühlsystem zudem einen Mechanismus zum Messen eines biomedizinischen Signals und einen Mechanismus zum Übermitteln eines elektrischen Reizes umfasst, der mit einer von der zweiten Elektrodeneinheit (42, 42a), einer dritten Elektrodeneinheit (40, 40a) und dem zweiten zylindrischen Körper (12) verbunden ist, wobei der zweite zylindrische Körper (12) von dem ersten zylindrischen Körper (14) oder der Umgebung elektrisch isoliert in Längsrichtung verläuft.

## Revendications

1. Système de refroidissement (22) comprenant :
une sonde aiguille de refroidissement (10, 10a) comprenant un premier corps cylindrique (14) et un deuxième corps cylindrique (12) qui sont réalisés à partir de métaux dissimilaires à paroi pince et s'étendent longitudinalement en étant électriquement isolés l'un de l'autre, où le deuxième corps cylindrique (12) est installé autour du premier corps cylindrique (14), où le premier corps cylindrique (14) et le deuxième corps cylindrique (12) sont fermés et reliés à une extrémité, en formant une partie distale (18) qui interconnecte électriquement le premier corps cylindrique (14) et le deuxième corps cylindrique (12) ; une section de communication minute (20) étant formée à proximité de la partie distale (18) pour communiquer entre un intérieur et un extérieur du premier corps cylindrique (14) ; où
la sonde aiguille de refroidissement (10, 10a) est configurée de telle sorte qu'une réfrigérant s'écoulant dans une parmi : une cavité entre le deuxième corps cylindrique (12) et le premier corps cylindrique (14) ; et une cavité dans le premier corps cylindrique (14) s'évapore et se dilate de manière adiabatique lors du passage à travers la section de communication minute (20) en produisant ainsi le froid, qui s'écoule dans l'autre cavité pour refroidir la partie distale (18) ; et
la partie distale (18) sert de jonction de mesure d'un thermocouple tandis que le premier corps cylindrique (14) sert de conducteur de compensation du thermocouple,
le système de refroidissement (22) comprenant en outre :
une prise de sonde (10) installée sur un côté opposé à la partie distale (18) de la sonde aiguille de refroidissement (10, 10a) ; une amenée de réfrigérant et un mécanisme d'évacuation (26) aptes à amener un réfrigérant à la sonde aiguille de refroidissement (10, 10a) et à évacuer le réfrigérant après que le froid a été libéré ; et un mécanisme de commande (28) de la quantité de réfrigérant apte à commander une quantité d'amenée du réfrigérant sur la base d'un signal provenant du thermocouple,
**caractérisé en ce que** le deuxième corps cylindrique (12) sert comme autre conducteur de compensation du thermocouple, et
le mécanisme de commande (28) de la quantité de réfrigérant est équipé d'une jonction de référence de thermocouple connectée aux extrémités respectives du premier corps cylindrique et du deuxième corps cylindrique.

2. Système de refroidissement (22) selon la revendication 1, **caractérisé en ce qu'**une combinaison de matériaux pour le premier corps cylindrique (14) et le deuxième corps cylindrique (12) est sélectionnée parmi une combinaison de Chromel et Alumel, une combinaison de Chromel et de cuivre, une combinaison de Chromel et de Constantan, une combinaison d'acier inoxydable de grade 304 et de Kovar, une combinaison de Constantan et de cuivre et une combinaison de platine et de platine-rhodium.

3. Système de refroidissement (22) selon la revendication 1, **caractérisé en ce que** la sonde a un diamètre de 1mm ou plus petit.

4. Système de refroidissement (22) selon la revendication 1, **caractérisé en ce que** le deuxième corps cylindrique (12) qui s'étend longitudinalement en étant électriquement isolé du premier corps cylindrique (14) ou de l'environnement est connecté à un mécanisme de mesure de signal biomédical ou un mécanisme de fourniture de stimulus électrique pour pouvoir mesurer un signal biomédical ou pour fournir un stimulus électrique à un corps vivant, via la partie distale (18).

5. Système de refroidissement (22) selon la revendication 1, dans lequel la sonde aiguille de refroidissement (10, 10a) comprend en outre une première unité d'électrode (36) qui s'étend longitudinalement en étant électriquement isolée de l'environnement et possède une pointe dans ou près de la partie distale (18), **caractérisé en ce que** la première unité d'électrode (36) est apte à mesurer un signal biomédical via la pointe et/ou fournir un stimulus électrique à un corps vivant via la pointe en étant connectée à un mécanisme de mesure de signal biomédical ou un mécanisme de fourniture de stimulus électrique ou étant connectée au mécanisme de mesure de signal biomédical et au mécanisme de fourniture de stimulus électrique de manière à pouvoir commuter entre le mécanisme de mesure de signal biomédical et le mécanisme de fourniture de stimulus électrique.

6. Système de refroidissement (22) selon la revendication 1, dans lequel la sonde aiguille de refroidissement (10, 10a) comprend en outre une première unité d'électrode (36) qui s'étend longitudinalement en étant électriquement isolée de l'environnement et possède une pointe dans ou près de la partie distale (18), **caractérisé en ce qu'**un parmi le deuxième corps cylindrique (12) qui s'étend longitudinalement en étant électriquement isolé du premier corps cylindrique (14) ou de l'environnement et la première unité d'électrode (36) est apte à mesurer un signal biomédical d'une zone prédéterminée d'un corps vivant et/ou fournir un stimulus électrique à la zone prédéterminée du corps vivant, via la partie distale (18) ou la pointe, en étant connecté à un mécanisme de mesure de signal biomédical ou un mécanisme de fourniture de stimulus électrique ou en étant connecté au mécanisme de mesure de signal biomédical et au mécanisme de fourniture de stimulus électrique de manière à permettre une commutation entre le mécanisme de mesure de signal biomédical et le mécanisme de fourniture de stimulus électrique, et l'autre des deux peut mesurer un signal biomédical d'une zone autre que la zone prédéterminée en étant connecté au mécanisme de mesure de signal biomédical.

7. Système de refroidissement (22) selon la revendication 1, dans lequel la sonde aiguille de refroidissement (10, 10a) comprend en outre une deuxième unité d'électrode (42, 42a) et une troisième unité d'électrode (40, 40a) dont chacune s'étend longitudinalement en étant électriquement isolée de l'environnement et possède une pointe dans ou près de la partie distale (18), **caractérisé en ce que** la deuxième unité d'électrode (42, 42a) est apte à mesurer un signal biomédical via la pointe en étant connectée à un mécanisme de mesure de signal biomédical ; et la troisième unité d'électrode (40, 40a) est apte à fournir un stimulus électrique à un corps vivant via la pointe en étant connectée à un mécanisme de fourniture de stimulus électrique.

8. Système de refroidissement (22) selon la revendication 1, dans lequel la sonde aiguille de refroidissement (10, 10a) comprend en outre une deuxième unité d'électrode (42,42a) et une troisième unité d'électrode (40, 40a) dont chacune s'étend longitudinalement en étant électriquement isolée de l'environnement et possède une pointe dans ou près de la partie distale (18), **caractérisé en ce qu'**une parmi la deuxième unité d'électrode (42, 42a), la troisième unité d'électrode (40, 40a) et le deuxième corps cylindrique (12) est apte à mesurer un signal biomédical d'une zone prédéterminée d'un corps vivant via la pointe en étant connecté à un mécanisme de mesure de signal biomédical, où le deuxième corps cylindrique (12) s'étend longitudinalement en étant électriquement isolé du premier corps cylindrique (14) ou de l'environnement ; un autre des trois est apte à fournir un stimulus électrique à la zone prédéterminée du corps vivant via la pointe en étant connecté à un mécanisme de fourniture de stimulus électrique ; et celui qui reste est apte à mesurer un signal biomédical d'une zone autre que la zone prédéterminé.

9. Système de refroidissement (22) selon la revendication 4, où le système de refroidissement comprend en outre un mécanisme de mesure de signal biomédical ou un mécanisme d'amenée de stimulus électrique connecté au premier corps cylindrique (14) ou au deuxième corps cylindrique (12).

10. Système de refroidissement (22) selon la revendication 5, où le système de refroidissement comprend en outre un mécanisme de mesure de signal biomédical et/ou un mécanisme de fourniture de stimulus électrique connecté à la première unité d'électrode (36).

11. Système de refroidissement (22) selon la revendication 6, dans lequel le système de refroidissement comprend en outre un mécanisme de mesure de signal biomédical et/ou un mécanisme de fourniture de stimulus électrique connecté à l'un parmi le deuxième corps cylindrique (12) et la première unité d'électrode (36), où le deuxième corps cylindrique (12) s'étend longitudinalement en étant électriquement isolé du premier corps cylindrique (14) ou de l'environnement.

12. Système de refroidissement (22) selon la revendication 7, dans lequel le système de refroidissement comprend en outre un mécanisme de mesure de signal biomédical connecté à la deuxième unité d'électrode (42, 42a) ; et un mécanisme de fourniture de stimulus électrique connecté à une troisième unité d'électrode (40, 40a).

13. Système de refroidissement (22) selon la revendication 8, où le système de refroidissement comprend en outre un mécanisme de mesure de signal biomédical et un mécanisme de fourniture de stimulus électrique connecté à un parmi la deuxième unité d'électrode (42, 42a), une troisième unité d'électrode (40, 40a) et le deuxième corps cylindrique (12), où le deuxième corps cylindrique (12) s'étend longitudinalement en étant électriquement isolé du premier corps cylindrique (14) ou de l'environnement.
